# FASCICULE DE BREVET EUROPEEN

(11) **EP 0 914 195 B1**
(45) Date de publication et mention de la délivrance du brevet: **12.06.2002**
(21) Numéro de dépôt: 97919434.7
(22) Date de dépôt: 27.03.1997
(51) Int. Cl.: B01D 61/44, C07C 51/31, C07C 51/42, C07C 55/14, B01J 38/00, B01J 23/92

(54) **PROCEDE DE SEPARATION D'UN CATALYSEUR PAR ELECTRODIALYSE MEMBRANAIRE**
VERFAHREN ZUR ABTRENNUNG EINES KATALYSATORS DURCH MEMBRAN-ELEKTRODIALYSE
METHOD FOR SEPARATING A CATALYST BY MEMBRANE ELECTRODIALYSIS

(30) Priorité: 02.04.1996 FR 9604379
(43) Date de publication de la demande: 12.05.1999
(73) Titulaire: RHODIA POLYAMIDE INTERMEDIATES, 69190 Saint-Fons (FR)
(72) Inventeur: FACHE, Eric, F-69100 Villeurbanne (FR); HORBEZ, Dominique, F-95130 Franconville (FR); LECONTE, Philippe, F-69330 Meyzieu (FR)
(74) Mandataire: Esson, Jean-Pierre
(86) Numéro de dépôt international: FR9700559
(87) Numéro de publication internationale: WO9736673

(56) Documents cités:
- DE-A- 1 954 707
- DE-A- 1 963 101
- FR-A- 2 722 783
- US-A- 4 680 098
- DESALINATION, vol. 79, no. 2 / 03, 1 Décembre 1990, pages 233-247, XP000244545 SRIDHAR S: "DESALINATION AND RECOVERY OF CATALYSTS BY ELECTRODIALYSIS"

## Description

La présente invention concerne un procédé de séparation par électrodialyse membranaire d'un catalyseur à partir d'une solution le contenant.

Plus précisément, elle concerne la séparation d'un catalyseur mis en oeuvre dans une réaction d'oxydation par l'oxygène moléculaire en phase homogène.

Les procédés d'oxydation en catalyse homogène sont relativement nombreux. Ainsi l'oxydation des cycloalcanes en diacides correspondants peut être réalisée en utilisant un sel soluble de métaux lourds comme le cobalt ou le manganèse.

Le brevet US 2 223 493, publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt. Ce brevet prévoit un séparation de l'acide adipique formé par cristallisation, mais n'enseigne rien sur la manière de recycler le catalyseur, dans une nouvelle opération d'oxydation, ni a fortiori sur l'activité qu'aurait un catalyseur recyclé une ou plusieurs fois.

Le brevet FR-A-2 722 783 décrit un procédé de séparation et de recyclage d'un catalyseur au cobalt, ayant servi pour l'oxydation du cyclohexane en acide adipique, après séparation du milieu réactionnel des principaux produits de la réaction et d'au moins une partie du solvant acide acétique. Ce procédé consiste essentiellement à extraire la majorité du catalyseur à l'aide de cyclohexane ou d'un mélange de cyclohexane et d'acide acétique. Ce procédé est efficace et le catalyseur recyclé n'a pas perdu de son activité. Cependant il fait intervenir des quantités importantes de solvant et nécessite plusieurs opérations successives.

Il apparaît donc souhaitable de disposer d'un procédé de séparation d'un catalyseur homogène dissous dans un milieu réactionnel, qui soit aussi efficace tout en étant de mise en oeuvre plus simple.

Le brevet US 4,680,098 décrit un procédé de récupération d'ions cobalt et manganèse, à partir d'une solution aqueuse diluée contenant également des impuretés, par séparation dans un électrodialyseur composé d'unités ayant chacune trois compartiments.

Le brevet FR-A-1 591 176 décrit un procédé de récupération des catalyseurs métalliques et de l'acide nitrique présents dans les eaux-mères résultant de la séparation de la masse réactionnelle obtenue lors de l'oxydation nitrique de cyclohexanol et/ou de cyclohexanone, consistant à faire passer une partie des eaux-mères contenant les sels métalliques, l'acide nitrique et les acides organiques dans une cellule d'électrodialyse. Les catalyseurs métalliques utilisés sont des sels de cuivre ou de vanadium.

Le brevet FR-A-2 026 288 décrit un procédé de récupération d'une partie importante d'acide nitrique et d'ions métalliques d'un liquide résiduel acide produit lors de la fabrication d'acide adipique par oxydation de la cyclohexanone ou du cyclohexanol en phase liquide, comprenant l'introduction de ce liquide dans un dispositif d'électrodialyse consistant en un ou plusieurs électrodialyseurs, pour récupérer l'acide nitrique et les ions métalliques dans un liquide récupérateur qui peut être de l'eau ou une solution diluée d'acide nitrique. Les catalyseurs métalliques utilisés sont des sels de cuivre ou de vanadium.

Ces deux procédés sont très semblables, voire identiques, et mettent en oeuvre des solutions contenant de fortes concentrations en acide nitrique. Cette particularité favorise considérablement la séparation des sels métalliques, sous forme de nitrates, des acides carboxyliques non disssociés.

La présente invention, telle que définie dans la revendication 1, concerne la séparation d'un catalyseur homogène mis en oeuvre dans l'oxydation du cyclohexane par l'oxygène et ne comportant donc pas d'acide nitrique. Elle concerne plus précisément un procédé de séparation d'un catalyseur homogène dissous dans un milieu contenant aussi au moins un diacide aliphatique et issu d'une réaction d'oxydation des cycloalcanes par l'oxygène moléculaire, le catalyseur contenant du cobalt, caractérisé en ce que la séparation est effectuée par électrodialyse membranaire dans un appareil d'électrodialyse qui comporte des cellules à deux compartiments.

Les catalyseurs homogènes sont les composés des métaux habituellement utilisés pour l'oxydation des cycloalcanes en diacides aliphatiques. Ce sont plus particulièrement les catalyseurs contenant du cobalt, seul ou avec d'autres métaux tels que le manganèse, le cuivre, le fer, le vanadium, le cérium ou les mélanges de ces métaux. Ces métaux sont sous forme de composés solubles dans le milieu réactionnel d'oxydation des cycloalcanes. De tels composés sont les hydroxydes, les oxydes, les sels organiques ou minéraux. Les composés préférés sont les sels de cobalt, seuls ou associés à d'autres composés à base de métaux tels que le manganèse et/ou le cuivre et/ou le fer et/ou le cérium et/ou le vanadium.

A titre d'exemples de ces sels de cobalt, on peut citer le chlorure de cobalt, le bromure de cobalt, le nitrate de cobalt, les carboxylates de cobalt comme l'acétate de cobalt, le propionate de cobalt, l'adipate de cobalt, le glutarate de cobalt, le succinate de cobalt. L'un des solvants utilisés le plus souvent pour l'oxydation des cycloalcanes étant l'acide acétique, l'acétate de cobalt tétrahydraté est particulièrement préféré.

Le mélange soumis à l'électrodialyse membranaire contient au moins un diacide formé lors de l'oxydation du cycloalcane et fréquemment un ou plusieurs autres diacides également formés comme sous-produits. Il peut également contenir l'ensemble des sous-produits de la réaction. Lorsque le catalyseur est utilisé pour l'oxydation du cyclohexane, on obtient majoritairement de l'acide adipique, mais également de l'acide glutarique et de l'acide succinique, ainsi que des quantités plus ou moins importantes de cyclohexanol, de cyclohexanone, d'esters de cyclohexyle, de lactones, d'acides hydroxycarboxyliques.

Bien que la réaction d'oxydation soit généralement réalisée dans un solvant organique, de préférence l'acide acétique pour l'oxydation du cyclohexane, ou le cas échéant sans solvant, le mélange à électrodialyser contient de préférence de l'eau.

Le milieu dans lequel se trouve le catalyseur homogène comprend donc préférentiellement de l'eau, le solvant ayant éventuellement servi dans le procédé qui a conduit à la solution à traiter pouvant être entièrement ou partiellement remplacé par l'eau avant l'électrodialyse. Généralement l'eau représente de 10 % à 100 % du milieu solvant de la solution soumise à l'électrodialyse et de préférence de 50 % à 100 % de ce milieu solvant.

Schématiquement, l'électrodialyse est un procédé qui permet, sous l'influence d'un champ électrique continu, d'extraire par migration au travers de membranes échangeuses d'ions les espèces ionisées contenues dans la solution à traiter.

L'appareil d'électrodialyse mis en oeuvre est constitué par différents compartiments délimités alternativement par des membranes cationiques et des membranes anioniques. Ces compartiments se divisent en compartiments de dilution (D) qui s'appauvrissent en composé à séparer, c'est-à-dire en catalyseur dans le procédé de l'invention, et en compartiments de concentration (C) qui à l'inverse s'enrichissent en composé à séparer.

En effet, sous l'action du champ électrique les cations de la solution à traiter migrent vers la cathode en sortant du compartiment (D) où ils se trouvent, à travers une membrane échangeuse de cations (membrane cationique). Lorsqu'ils sont passés dans le compartiment (C) suivant, ils ne peuvent pas le quitter en raison de la présence de la membrane échangeuse d'anions (membrane anionique) suivante. Simultanément les anions migrent vers l'anode en traversant une membrane anionique et passent dans un compartiment (C) adjacent, qu'ils ne peuvent ensuite pas quitter en raison de la présence de la membrane cationique suivante.

Deux compartiments (C) et (D) adjacents forment une cellule d'électrodialyse. Un électrodialyseur comporte un empilement de plusieurs cellules. Ce nombre de cellules par électrodialyseur est de manière générale aussi élevé que possible. Par exemple, ce nombre peut varier avantageusement entre 10 et 500 cellules.

En pratique, les membranes anioniques et cationiques sont disposées alternativement dans un système de type filtre-presse.

Les membranes homopolaires utilisées dans le procédé de l'invention se divisent en deux grandes familles, selon leur mode de fabrication.

Ce sont tout d'abord les membranes hétérogènes, préparées à partir de résines échangeuses d'ions, mélangées à un liant tel que polychlorure de vinyle, polyéthylène ou autre. L'ensemble ainsi formé peut enduire une trame comme par exemple un tissu de polyester ou de polyacrylonitrile.

Ce sont également les membranes homogènes, obtenues par introduction d'un groupement fonctionnel sur un support inerte, par greffage chimique ou radiochimique. La méthode chimique, la plus utilisée, consiste généralement à fonctionnaliser un latex d'un polymère comportant des noyaux aromatiques, tel que styrène/divinylbenzène ou styrène/butadiène. Le latex ainsi fonctionnalisé sert ensuite à enduire une trame comme pour les membranes hétérogènes. La méthode radiochimique comporte généralement le greffage, sous l'influence d'un rayonnement, d'un composé aromatique, tel que le styrène, sur un support inerte comme une feuille de polyéthylène ou de polytétrafluoroéthylène. Le noyau aromatique est ensuite fonctionnalisé comme dans la méthode chimique.

Les membranes échangeuses de cations (membranes cationiques) comportent des groupements acides forts, le plus souvent des groupements sulfonates, ou des groupements acides faibles, souvent des groupements carboxylates. Plus rarement les groupements acides peuvent être des groupements PO₃²⁻, HPO₂⁻, AsO₃²⁻, SeO₃⁻.

Les membranes échangeuses d'anions (membranes anioniques) comportent des groupement basiques forts, le plus souvent des groupements ammonium quaternaire, ou des groupements basiques faibles, le plus souvent des groupements amines. Plus rarement les groupements basiques peuvent être des groupements phosphonium quaternaire ou des groupements sulfonium.

Dans le présent procédé, les membranes cationiques comportent de préférence des groupements acides forts et parmi ceux-ci préférentiellement des groupements sulfonates et les membranes anioniques comportent de préférence des groupements basiques forts et parmi ceux-ci préférentiellement des groupements ammonium quaternaire.

Outre les membranes, l'électrodialyseur comporte bien entendu une cathode et une anode. L'anode est constituée en des matériaux classiquement utilisés en électrodialyse, par exemple en graphite, en titane revêtu par des métaux précieux ou des oxydes de métaux précieux, notamment le titane platiné. La cathode est également constituée en des matériaux classiquement utilisés en électrodialyse, par exemple en graphite, en acier inoxydable, en nickel.

L'électrodialyseur est alimenté avec la solution au moins partiellement aqueuse à traiter. Il est également nécessaire de faire circuler à l'anode une solution d'un anolyte et à la cathode une solution d'un catholyte. Ces solutions constituent souvent une solution unique d'électrolyte. Dans le présent procédé, un circuit unique d'électrolyte convient bien. Le rôle de la solution d'électrolyte est d'assurer une conductivité suffisante. De préférence, cette conductivité sera égale ou supérieure à 20 millisiemens par centimètre (mS/cm), sans que cette limite inférieure soit à considérer comme critique pour la mise en oeuvre du présent procédé.

L'électrolyte mis en oeuvre est un composé ionisable tel qu'un sel, un acide ou une base. L'électrolyte est de préférence choisi parmi les composés non électroactifs. Ainsi, par exemple, il est préférable de ne pas utiliser industriellement des chlorures qui généreraient du chlore à l'anode.

On peut citer comme exemples d'électrolytes, des sels neutres comme les sulfates, des acides comme l'acide sulfamique, les acides carboxyliques solubles dans l'eau, l'acide sulfurique. On peut également utiliser comme électrolyte un sel du métal catalyseur, plus particulièrement un sel de cobalt, tel que par exemple l'acétate de cobalt.

Dans le présent procédé, il faudra éviter d'utiliser des solutions d'électrolyte dont le pH pourrait conduire à la précipitation du composé métallique que l'on souhaite séparer par électrodialyse. C'est pourquoi, on choisira de préférence un électrolyte acide.

La tension appliquée à l'électrodialyseur doit être de nature à éviter la polarisation du système, c'est-à-dire une dissociation de l'eau sous l'effet d'un champ électrique trop intense. En général, une tension de 0,5 volt à 2,5 volt/cellule et de préférence de 0,5 volt à 1,5 volt/cellule est appropriée. L'effet de polarisation peut être diminué en augmentant la turbulence du liquide, par l'utilisation de cellules minces conjointement avec des cadres séparateurs. On préfère des cellules ayant une largeur de 0,5 mm à 2 mm et de préférence de 0,75 mm à 1,5 mm.

La température à laquelle est mis en oeuvre le procédé de l'invention se situe dans un domaine compatible avec la stabilité des membranes. En effet, si en principe les températures élevées sont favorables, en accroissant la mobilité électrolytique et en réduisant la viscosité de la solution à traiter, l'augmentation de la température diminue la durée de vie des membranes. On opérera donc de préférence à une température inférieure ou égale à 50°C et plus particulièrement entre 20°C et 40°C.

L'électrodialyseur peut fonctionner de différentes façons. Il peut tout d'abord fonctionner en continu (fonctionnement en passage direct), la solution à traiter traversant en continu l'empilement ; plusieurs étages sont alors disposés en série si le taux de traitement à obtenir l'exige. Il peut aussi fonctionner en discontinu (fonctionnement en recirculation), la solution à traiter recirculant sur une cuve jusqu'à ce que le taux de traitement souhaité soit obtenu. Enfin il peut fonctionner en passage direct avec une recirculation partielle.

Le mélange réactionnel, dans lequel se trouve le catalyseur homogène à séparer ainsi que les diacides, provient essentiellement, comme cela à été indiqué précédemment, des procédés d'oxydation des cycloalcanes en diacides correspondants. Pour simplifier, il sera question généralement dans la description qui suit de l'oxydation du cyclohexane en acide adipique, conduisant à la formation de quantités plus faibles, mais néanmoins importantes, d'acide glutarique et d'acide succinique.

Avant de traiter par électrodialyse un tel mélange, il est généralement avantageux de réaliser certaines opérations permettant en particulier de séparer la majeure partie de l'acide adipique, composé dont la préparation est visée.

Cette séparation peut être effectuée de manière connue, par exemple en précipitant l'acide adipique par refroidissement de ce mélange.

La solution restante est alors reprise par de l'eau, après éventuellement une élimination partielle ou totale du solvant organique qu'elle peut contenir, afin d'être soumise à l'électrodialyse selon l'invention.

Généralement la solution que l'on électrodialyse contient de 0,0001 mole à 1 mole de catalyseur par kilogramme, de 0,001 mole à 1 mole d'acide glutarique par kilogramme, de 0,001 mole à 1 mole d'acide succinique par kilogramme et de 0,001 mole à 1 mole d'acide adipique par kilogramme.

Les exemples qui suivent illustrent l'invention.

### EXEMPLE 1

L'électrodialyseur utilisé est constitué par un empilement de 10 cellules de 2 dm² de surface active, composées chacune d'un compartiment où l'on introduit la solution à traiter (compartiment D où ladite solution sera diluée en catalyseur) et d'un compartiment C qui recevra le catalyseur au cours de l'électrodialyse.

Les membranes séparant chaque compartiment D du compartiment C adjacent sont :
- membrane anionique de marque NEOSEPTA® AMX à groupements ammonium quaternaire,
- membrane cationique de marque NEOSEPTA® CMX à groupements sulfonates.

L'électrolyte est constitué par une solution aqueuse d'acide sulfamique, ayant une conductivité de 20 mS/cm à 20°C. Le débit de circulation de cette solution est de 400 l/h et son volume est de 2 l.

La solution aqueuse à traiter a un volume de 1,6 I et elle contient au départ :
- 0,18 mol/kg d'acide adipique
- 0,87 mol/kg d'acide glutarique
- 0,36 mol/kg d'acide succinique
- 0,27 mol/kg de cobalt sous forme d'acétate de cobalt.

On a utilisé le mode de fonctionnement discontinu (fonctionnement en recirculation).

Le débit de circulation de cette solution dans les compartiments D est de 180 l/h.

La solution qui circule dans les compartiments C et qui va recevoir le sel de cobalt est au départ une solution aqueuse de chlorure de sodium à 5 g/l ; elle a un volume de 1,6 I et elle circule avec un débit de 180 l/h.

La conductivité initiale de chacune des solutions des compartiments C est de 10 mS/cm.

L'électrodialyse est conduite à tension imposée de 18 V.

Des échantillons des différentes solutions sont prélevés périodiquement afin de suivre l'évolution de l'opération. On dose le cobalt par absorption atomique et les diacides par chromatographie en phase vapeur. On suit également le pH, la conductivité et le volume des solutions.

Le tableau 1 ci-après rassemble les résultats des mesures concemant le pH, la conductivité et le volume des solutions.

Le tableau 2 ci-après rassemble les résultats concernant les concentrations en mol/kg des différents prélèvements. Par définition, L1 représente la solution des compartiments D, aussi appelée solution alimentaire, L2 représente la solution des compartiments C, aussi appelée solution concentrée et L3 représente la solution de l'électrolyte.

Comme cela peut être constaté dans les résultats du tableau 1, la solution alimentaire s'appauvrit en eau ; cela s'explique par le fait que les ions qui migrent sont hydratés et qu'en fin d'essai la différence de conductivité entre la solution alimentaire et la solution concentrée est importante (phénomène d'osmose).

Pour établir des bilans précis, on exprimera dans le tableau 2 les résultats en tenant compte des variations de volume. Les résultats du tableau 2 sont exprimés en considérant que l'on part de 1 kg de flux à traiter.

Le symbole "-" dans le tableau signifie que la mesure n'a pas été effectuée.

**Tableau 1**

| **Temps en min** | **Solution prélevée** | **Température en °C** | **pH** | **Conductivité en mS/cm** | **Volume en ml** |
|---|---|---|---|---|---|
| 0 | L1 | 20,0 | 3,5 | 10,7 | 1600 |
| 0 | L2 | 20,0 | 4,6 | 10,1 | 1600 |
| 0 | L3 | 20,0 | - | 20,0 | 2000 |
| 18 | L1 | - | 3,5 | 9,6 | 1567 |
| 20 | L2 | - | 4,7 | 14,1 | 1639 |
| 38 | L1 | - | 3,2 | 7,6 | 1530 |
| 40 | L2 | 27,2 | 4,7 | 16,4 | 1677 |
| 57 | L1 | 28,4 | 2,9 | 5,1 | 1495 |
| 58 | L2 | - | 4,9 | 17,8 | 1712 |
| 80 | L1 | - | 2,1 | 1,9 | 1416 |
| 80 | L2 | - | 4,8 | 18,8 | 1793 |
| 86 | L1 | 30,2 | 2,0 | 1,8 | 1405 |
| 86 | L2 | - | 4,8 | 18,9 | 1805 |

**Tableau 2**

| **Temps en min** | **Solution prélevée** | **Co en mol/kg** | **Acide adipique en mol/kg** | **Acide glutarique en mol/kg** | **Acide succinique en mol/kg** |
|---|---|---|---|---|---|
| 0 | L1 | 0,267 | 0,183 | 0,869 | 0,355 |
| 0 | L2 | 0 | 0 | 0 | 0 |
| 0 | L3 | 0 | 0 | 0 | 0 |
| 0 | Total | 0,267 | 0,183 | 0,869 | 0,355 |
| 18 | L1 | 0,192 | 0,184 | 0,867 | 0,340 |
| 20 | L2 | 0,066 | 0 | 0 | 0,002 |
| 20 | Total | 0,258 | 0,184 | 0,867 | 0,342 |
| 38 | L1 | 0,137 | 0,175 | 0,822 | 0,316 |
| 40 | L2 | 0,127 | 0,005 | 0,050 | 0,036 |
| 40 | Total | 0,264 | 0,180 | 0,872 | 0,352 |
| 57 | L1 | 0,064 | 0,174 | 0,827 | 0,306 |
| 58 | L2 | 0,193 | 0 | 0,044 | 0,076 |
| 58 | Total | 0,257 | 0,174 | 0,871 | 0,382 |
| 80 | L1 | 0,005 | 0,149 | 0,753 | 0,271 |
| 80 | L2 | 0,251 | 0 | 0,082 | 0,080 |
| 80 | Total | 0,256 | 0,149 | 0,835 | 0,351 |
| 86 | L1 | 0,0005 | 0,163 | 0,774 | 0,269 |
| 86 | L2 | 0,244 | 0 | 0,081 | 0,078 |
| 86 | L3 | 0,001 | 0 | 0 | 0,004 |
| 86 | Total | 0,245 | 0,163 | 0,855 | 0,351 |

### EXEMPLE 2

### Oxydation du cyclohexane en acide adipique

Cet exemple a pour but de préparer une solution à mettre en oeuvre dans le procédé de séparation du catalyseur par électrodialyse.

Dans un autoclave de 1,5 litre chemisé en titane et équipé d'une turbine et de diverses ouvertures pour l'introduction des réactifs et des fluides ou pour l'évacuation des produits de la réaction et des fluides, on charge à température ambiante, après avoir préalablement purgé l'appareillage avec de l'azote :
- acétate de Co tétrahydraté : 4,0 g
- acide acétique : 359 g
- cyclohexane : 289,7 g
- acétaldéhyde : 1,2 g

Après fermeture de l'autoclave, la pression d'azote est portée à 20 bar (2 MPa), l'agitation est mise en route (800 tours/min) et la température est amenée à 120°C en 29 minutes. L'azote est alors remplacé par 20 bar d'air à 4 % d'oxygène. Le débit gazeux de sortie est réglé à 250 litres/heure.

Après une période d'induction de 10 min, pendant laquelle il n'y a pas de consommation d'oxygène, la température s'élève brutalement à 106°C et l'oxygène commence à être consommé. A l'aide d'un système de débitmètres, on amène la teneur en oxygène de l'air à l'entrée à 16 %. La teneur en oxygène à la sortie de l'autoclave reste inférieure à 5 % durant la totalité de l'essai. La température moyenne dans l'autoclave est maintenue à 106-107°C.

Lorsque 50 litres d'oxygène ont été consommés (ce qui correspond à un taux de transformation du cyclohexane d'environ 20 %), on injecte en continu du cyclohexane (4,3 ml/min) et une solution acétique d'acétate de Co tétrahydraté à 1,1 % en poids par poids (débit de 3,9 ml/min).

L'injection est poursuivie jusqu'à l'obtention d'un mélange réactionnel comprenant environ 5700 g d'acide adipique et constitué d'une phase acétique de 35,3 kg et d'une phase cyclohexanique de 10,3 kg.

Le niveau du liquide dans l'autoclave est maintenu constant à l'aide d'une sonde de niveau. Le mélange réactionnel est récupéré dans un récipient en verre chauffé à 70C, grace à une vanne pneumatique de fond asservie.

La séparation des deux phases du mélange réactionnel obtenu est effectuée à 70°C.

La phase acétique est concentrée jusqu'à une masse de 19 kg environ. L'acide adipique cristallise et il est séparé par filtration. Il est recristallisé dans l'eau (on obtient ainsi 4,2 kg d'acide adipique purifié).

Le mélange des solutions acétique et aqueuse provenant de la cristallisation et de la recristallisation de l'acide adipique représente environ 11,5 kg. Ce mélange est concentré à environ 50 % de sa masse initiale, puis est dilué par environ 2 fois sa masse d'eau. Par décantation, on élimine une partie des composés cyclohexanone, cyclohexanol, esters.

On obtient ainsi une solution hydroacétique de composition suivante :
- cobalt 0,4485 % en poids par poids
- acide acétique 193 g par kg de solution
- eau 626 g par kg de solution
- acide adipique 41 g par kg de solution
- acide glutarique 27,9 g par kg de solution
- acide succinique 13,3 g par kg de solution
- acide hydroxycaproïque 4,8 g par kg de solution
- acide hydroxyadipique 9,4 g par kg de solution
- cyclohexanone 10,6 g par kg de solution
- cyclohexanol 5,7 g par kg de solution
- acétate de cyclohexyle 3,4 g par kg de solution
- butyrolactone 6,4 g par kg de solution
- valérolactone 0,8 g par kg de solution
- esters divers de cyclohexyle 41,2 mmol par kg de solution.

### EXEMPLE 3

L'électrodialyseur utilisé est constitué par un empilement de 10 cellules de 2 dm² de surface active, composées chacune d'un compartiment où l'on introduit la solution hydroacétique à traiter préparée dans l'exemple 2 (compartiment D où ladite solution sera diluée en catalyseur) et d'un compartiment C qui recevra le catalyseur au cobalt au cours de l'électrodialyse.

Les membranes séparant chaque compartiment D du compartiment C adjacent sont :
- membrane anionique de marque NEOSEPTA AMX à groupements ammonium quaternaire,
- membrane cationique de marque NEOSEPTA CMX à groupements sulfonates.

L'électrolyte est constitué par une solution aqueuse d'acide sulfamique, ayant une conductivité de 20 mS/cm à 20°C. Le débit de circulation de cette solution est de 400 l/h et son volume est de 2 l.

La solution hydroacétique à traiter a un volume de 1,6 I (composition indiquée dans l'exemple 2).

On a utilisé le mode de fonctionnement discontinu (fonctionnement en recirculation).

Le débit de circulation de cette solution dans les compartiments D est de 180 l/h.

La solution qui circule dans les compartiments C et qui va recevoir le sel de cobalt est au départ une solution aqueuse d'acétate de Co tétrahydraté à 10 g/l, pour avoir une conductivité initiale de 3,5 à 4 mS/cm ; elle a un volume de 1,6 I et elle circule avec un débit de 180 l/h.

L'électrodialyse est conduite à tension imposée de 18 V.

Des échantillons des différentes solutions sont prélevés périodiquement afin de suivre l'évolution de l'opération. On dose le cobalt par absorption atomique et les diacides et des autres composés organiques par chromatographie en phase vapeur. On suit également le pH, la conductivité et le volume des solutions.

Comme cela a déjà été signalé dans l'exemple 1, la solution alimentaire s'appauvrit en eau ; cela s'explique par le fait que les ions qui migrent sont hydratés et qu'en fin d'essai la différence de conductivité entre la solution alimentaire et la solution concentrée est importante (phénomène d'osmose).

Pour établir des bilans précis, on exprimera dans les tableaux 3 et 4 les résultats en tenant compte des variations de volume. Les résultats sont exprimés en considérant que l'on part de 1 kg de flux à traiter.

Le tableau 3 rassemble les résultats relatifs au catalyseur Co ; le tableau 4 rassemble les résultats relatifs aux composés organiques présents dans la solution à traiter.

**Tableau 3**

| **Temps en min** | **Solution prélevée** | **Co en mol/kg** | **Co %/Co initial dans L1** |
|---|---|---|---|
| 0 | L1 | 0,070 | 100 |
| 0 | L2 | 0,026 | 0 |
| 0 | L3 | 0 | 0 |
| 0 | Total | 0,096 | |
| 8 | L1 | 0,037 | 53 |
| 9 | L2 | 0,043 | 24,5 |
| 17 | L1 | 0,017 | 24,5 |
| 18 | L2 | 0,061 | 50 |
| 25 | L1 | 0,004 | 6 |
| 26 | L2 | 0,075 | 70 |
| 28 | L1 | 0,002 | 3 |
| 28 | L2 | 0,088 | 88,5 |
| 28 | L3 | 0,006 | 8,5 |
| 28 | Total | 0,096 | |

Cet essai sur mélange issu d'un essai d'oxydation du cyclohexane en acide adipique confirme l'efficacité de la séparation par électrodialyse du catalyseur Co.

**Tableau 4**

| **Composés** | **% restant par rapport à la solution L1 initiale** | | |
|---|---|---|---|
| | **temps 0 min** | **temps 17 min** | **temps 28 min** |
| acide adipique | 100 | 97,5 | 98,5 |
| acide glutarique | 100 | 95 | 93,5 |
| acide succinique | 100 | 91,5 | 88 |
| acide hydroxycaproïque | 100 | 100 | 100 |
| acide hydroxyadipique | 100 | 89 | 88 |
| cyclohexanol | 100 | 96 | 100 |
| acétate de cyclohexyle | 100 | 91 | 97,5 |
| cyclohexanone | 100 | 97 | 98 |
| butyrolactone | 100 | 91 | 79 |
| autres esters de cyclohexyle | 100 | 100 | 100 |
| acide acétique | 100 | 96 | 96 |

Les composés organiques restent très majoritairement dans la solution alimentaire.

### EXEMPLE 4

L'électrodialyseur utilisé est le même que pour l'exemple 3 et la solution hydroacétique à traiter est celle qui a été préparée dans l'exemple 2.

La solution qui circule dans les compartiments C et qui va recevoir le sel de cobalt est au départ la solution L2 obtenue dans un essai antérieur d'électrodialyse d'une partie de la solution préparée dans l'exemple 2 et la solution d'électrolyte L3 provient également de ce même essai antérieur.

Toutes les conditions de mise en oeuvre sont celles de l'exemple 3.

Le but de cet essai est de montrer que l'on peut enrichir progressivement la solution concentrée des compartiments C en Co.

Comme cela a déjà été signalé dans l'exemple 1, la solution alimentaire s'appauvrit en eau ; cela s'explique par le fait que les ions qui migrent sont hydratés et qu'en fin d'essai la différence de conductivité entre la solution alimentaire et la solution concentrée est importante (phénomène d'osmose).

Pour établir des bilans précis, on exprimera dans les tableaux 5 et 6 les résultats en tenant compte des variations de volume. Les résultats sont exprimés en considérant que l'on part de 1 kg de flux à traiter.

Le tableau 5 rassemble les résultats relatifs au catalyseur Co ; le tableau 6 rassemble les résultats relatifs aux composés organiques présents dans la solution à traiter.

**Tableau 5**

| **Temps en min** | **Solution prélevée** | **Co en mol/kg** | **Co %/Co initial dans L1** |
|---|---|---|---|
| 0 | L1 | 0,075 | 100 |
| 0 | L2 | 0,086 | 0 |
| 0 | L3 | 0,010 | 0 |
| 0 | Total | 0,171 | |
| 7 | L1 | 0,045 | 60 |
| 8 | L2 | 0,098 | 15,5 |
| 16 | L1 | 0,032 | 43 |
| 17 | L2 | 0,120 | 46 |
| 25 | L1 | 0,012 | 16 |
| 26 | L2 | 0,133 | 63 |
| 30 | L1 | 0,005 | 7 |
| 30 | L2 | 0,153 | 89 |
| 30 | L3 | 0,017 | 9 |
| 30 | Total | 0,175 | |

Cet exemple avec la mise en oeuvre d'une solution déjà concentrée en Co dans les compartiments C confirme l'efficacité de la séparation par électrodialyse du catalyseur Co et la possibilité d'obtenir des solutions encore plus concentrées en Co.

**Tableau 6**

| **Composés** | **% restant par rapport à la solution L1 initiale** | | |
|---|---|---|---|
| | **temps 0 min** | **temps 16 min** | **temps 30 min** |
| acide adipique | 100 | 104 | 95 |
| acide glutarique | 100 | 99,5 | 91,5 |
| acide succinique | 100 | 95 | 83,5 |
| acide hydroxycaproïque | 100 | 100 | 99 |
| acide hydroxyadipique | 100 | 100 | 88 |
| cyclohexanol | 100 | 99 | 100 |
| acétate de cyclohexyle | 100 | 92,5 | 100 |
| cyclohexanone | 100 | 97 | 100 |
| butyrolactone | 100 | 100 | 100 |
| autres esters de cyclohexyle | 100 | 100 | 93 |
| acide acétique | 100 | 95 | 95 |

## Revendications

1. Procédé de séparation d'un catalyseur métallique homogène dissous dans un milieu contenant aussi au moins un diacide aliphatique et issu d'une réaction d'oxydation des cycloalcanes par l'oxygène moléculaire, le catalyseur contenant du cobalt, **caracterisé en ce que** la séparation est effectuée par électrodialyse membranaire dans un appareil d'électrodialyse qui comporte un empilement de plusieurs cellules, chaque cellule étant constituée par deux compartiments de concentration (C) et de dilution (D) adjacents délimités alternativement par de membranes cationiques et des membranes anioniques.

2. Procédé selon la revendication 1, **caractérisé en ce que** le catalyseur est choisi parmi les catalyseurs contenant du cobalt, seul ou avec d'autres métaux tels que le manganèse, le cuivre, le fer, le vanadium, le cérium ou les mélanges de ces métaux.

3. Procédé selon la revendication 2, **caractérisé en ce que** les métaux sont sous forme de composés solubles dans le milieu réactionnel d'oxydation des cycloalcanes, tels que les hydroxydes, les oxydes, les sels organiques ou minéraux.

4. Procédé selon l'une des revendications 1 à 3, **caractérisé en ce que** le catalyseur est choisi parmi les sels de cobalt, seuls ou associés à d'autres composés à ' base de métaux tels que le manganèse et/ou le cuivre et/ou le fer et/ou le cérium et/ou le vanadium.

5. Procédé selon l'une des revendications 1 à 4, **caractérisé en ce que** le mélange soumis à l'électrodialyse membranaire contient au moins un diacide formé lors de l'oxydation du cycloalcane et un ou plusieurs autres diacides également formés comme sous-produits, de préférence de l'acide adipique et également de l'acide glutarique et de l'acide succinique.

6. Procédé selon l'une des revendications 1 à 5, **caractérisé en ce que** le milieu dans lequel se trouve le catalyseur homogène comprend l'eau, le solvant ayant éventuellement servi dans le procédé qui a conduit à la solution à traiter pouvant être entièrement ou partiellement remplacé par l'eau avant l'électrodialyse.

7. Procédé selon la revendication 6, **caractérisé en ce que** l'eau représente de 10 % à 100 % du milieu solvant de la solution soumise à l'électrodialyse et de préférence de 50 % à 100 % de ce milieu solvant.

8. Procédé selon l'une des revendications 1 à 7, **caractérisé en ce que** les membranes sont constituées d'une matrice sur laquelle sont greffés des groupes fonctionnels, soit anioniques tels que des groupes sulfonates pour les membranes cationiques, soit cationiques tels que des groupes ammonium quaternaire pour les membranes anioniques.

9. Procédé selon l'une des revendications 1 à 8, **caractérisé en ce que** la solution que l'on électrodialyse contient de 0,0001 mole à 1 mole de catalyseur par kilogramme, de 0,001 mole à 1 mole d'acide glutarique par kilogramme, de 0,001 mole à 1 mole d'acide succinique par kilogramme et de 0,001 mole à 1 mole d'acide adipique par kilogramme.

## Claims

1. Process for the separation of a homogeneous metal catalyst dissolved in a medium also comprising at least one aliphatic diacid and resulting from a reaction for the oxidation of cycloalkanes by molecular oxygen, the catalyst comprising cobalt, **characterized in that** the separation is carried out by membrane electrodialysis in an electrodialysis device which comprises a stack of several cells, each cell being composed of two adjacent concentrating and diluting compartments, respectively (C) and (D), delimited alternately by cationic membranes and anionic membranes.

2. Process according to claim 1, **characterized in that** the catalyst is chosen from catalysts comprising cobalt, alone or with other metals, such as manganese, copper, iron, vanadium, cerium or mixtures of these metals.

3. Process according to claim 2, **characterized in that** the metals are in the form of compounds which are soluble in the reaction medium for the oxidation of cycloalkanes, such as organic or inorganic hydroxides, oxides or salts.

4. Process according to one of claims 1 to 3, **characterized in that** the catalyst is chosen from cobalt salts, alone or in combination with other compounds based on metals such as manganese and/or copper and/or iron and/or cerium and/or vanadium.

5. Process according to one of claims 1 to 4, **characterized in that** the mixture subjected to membrane electrodialysis comprises at least one diacid formed during the oxidation of the cycloalkane and one or more other diacids also formed as by-products, preferably adipic acid and also glutaric acid and succinic acid.

6. Process according to one of claims 1 to 5, **characterized in that** the medium in which the homogeneous catalyst is found comprises water, the solvent which has optionally been used in the process which has led to the solution to be treated being able to be entirely or partially replaced by water before electrodialysis.

7. Process according to claim 6, **characterized in that** water represents from 10% to 100% of the solvent medium of the solution subjected to electrodialysis and preferably from 50% to 100% of this solvent medium.

8. Process according to one of claims 1 to 7, **characterized in that** the membranes are composed of a matrix to which functional groups are grafted, either anionic functional groups, such as sulphonate groups, for cationic membranes or cationic functional groups, such as quaternary ammonium groups, for anionic membranes.

9. Process according to one of claims 1 to 8, **characterized in that** the solution which is subjected to electrodialysis comprises from 0.0001 mol to 1 mol of catalyst per kilogram, from 0.001 mol to 1 mol of glutaric acid per kilogram, from 0.001 mol to 1 mol of succinic acid per kilogram and from 0.001 mol to 1 mol of adipic acid per kilogram.

## Patentansprüche

1. Verfahren zur Abtrennung eines homogenen Metallkatalysators, der in einem Milieu gelöst ist, das zumindest eine aliphatische Disäure, hervorgegangen aus einer Oxidationsreaktion von Cycloalkanen mit molekularem Sauerstoff, enthält, wobei der Katalysator Kobalt enthält, **dadurch gekennzeichnet, daß** die Abtrennung durch Membran-Elektrodialyse in einer Elektrodialyseapparatur durchgeführt wird, die eine Abfolge mehrerer Zellen beinhaltet, wobei jede Zelle aus zwei benachbarten Abteilungen der Konzentration (C) und der Verdünnung (D) besteht, die abwechselnd von kationischen Membranen und anionischen Membranen abgegrenzt sind.

2. Verfahren gemäß Anspruch 1, **dadurch gekennzeichnet, daß** der Katalysator unter den Katalysatoren ausgewählt wird, die Kobalt, allein oder mit weiteren Metallen, wie Mangan, Kupfer, Eisen, Vanadin, Cer oder Gemische dieser Metalle, enthalten.

3. Verfahren gemäß Anspruch 2, **dadurch gekennzeichnet, daß** die Metalle in Form von Verbindungen vorliegen, die in dem Reaktionsmilieu der Oxidation der Cycloalkane löslich sind, wie die Hydroxide, die Oxide, die organischen oder anorganischen Salze.

4. Verfahren gemäß einem der Ansprüche 1 bis 3, **dadurch gekennzeichnet, daß** der Katalysator unter den Kobaltsalzen, allein oder assoziiert mit weiteren Verbindungen auf Basis von Metallen, wie Mangan und/oder Kupfer und/oder Eisen und/oder Cer und/oder Vanadin ausgewählt wird.

5. Verfahren gemäß einem der Ansprüche 1 bis 4, **dadurch gekennzeichnet, daß** das Gemisch, das der Membran-Elektrodialyse unterzogen wird, zumindest eine Disäure, die bei der Oxidation des Cycloalkans gebildet wird, und eine oder mehrere weitere Disäuren, die auch als Nebenprodukte gebildet werden, vorzugsweise Adipinsäure und auch Glutarsäure und Bernsteinsäure enthält.

6. Verfahren gemäß einem der Ansprüche 1 bis 5, **dadurch gekennzeichnet, daß** das Milieu, in dem sich der homogene Katalysator befindet, Wasser umfaßt, wobei das bei dem Verfahren, das zu der zu behandelnden Lösung geführt hat, gegebenenfalls gediente Lösungsmittel vor der Elektrodialyse vollständig oder teilweise durch Wasser ersetzt worden sein kann.

7. Verfahren gemäß Anspruch 6, **dadurch gekennzeichnet, daß** das Wasser 10 bis 100% des Lösungsmittelmilieus der Lösung, die der Elektrodialyse unterzogen wird, und vorzugsweise 50 bis 100% dieses Lösungsmittelmilieus ausmacht.

8. Verfahren gemäß einem der Ansprüche 1 bis 7, **dadurch gekennzeichnet, daß** die Membranen aus einer Matrix bestehen, auf der funktionelle Gruppen, entweder anionische, wie die Sulfonatgruppen für die kationischen Membranen, oder kationische, wie die quarternären Ammoniumgruppen für die anionischen Membranen, aufgepfropft sind.

9. Verfahren gemäß einem der Ansprüche 1 bis 8, **dadurch gekennzeichnet, daß** die Lösung, die man einer Elektrodialyse unterzieht, 0,0001 Mol bis 1 Mol Katalysator je kg, 0,001 Mol bis 1 Mol Glutarsäure je kg, 0,001 Mol bis 1 Mol Bernsteinsäure je kg und 0,001 Mol bis 1 Mol Adipinsäule je kg enthält.
